# EUROPEAN PATENT SPECIFICATION

(11) **EP 1 563 005 B1**
(45) Date of publication and mention of the grant of the patent: **29.03.2006**
(21) Application number: 03758083.4
(22) Date of filing: 30.10.2003
(51) Int. Cl.: C08K 5/3492, C07D 251/00

(54) **NEW CARBAMATE - MELAMINE FORMALDEHYDE CROSS-LINKING AGENTS**
NEUE CARBAMAT-MELAMIN-FORMALDEHYD VERNETZUNGSMITTEL
NOUVEAUX AGENTS RETICULANTS CARBAMATE-MELAMINE FORMALDEHYDE

(30) Priority: 08.11.2002 EP 02079718
(43) Date of publication of application: 17.08.2005
(73) Proprietor: Nuplex Resins B.V., 4612 RB Bergen op Zoom (NL)
(72) Inventor: SCHUTYSER, Jan, Andre, Jozef, NL-6952 JJ Dieren (NL); DE JONG, Aalbert, Johannes, NL-3781 XN Voorthuizen (NL)
(74) Representative: Derks, Wilbert
(86) International application number: PCT/EP2003/012070
(87) International publication number: WO 2004/041922

(56) References cited:
- EP-A- 0 624 577
- EP-A- 0 814 104
- EP-A- 0 850 986
- US-A- 4 710 542

## Description

The invention relates to carbamate-melamine formaldehyde cross-linking agents. Furthermore, the invention relates to the preparation of such cross-linking agents, to curable coating compositions comprising them, and to the use of these curable coating compositions.

Amino-1,3,5-triazine-derived compounds are often used as cross-linking agents in coating compositions comprising active hydrogen-containing resins. These types of cross-linkers are particularly desirable in coating compositions for automotive top coats because of their excellent durability, hardness, gloss, and appearance. Among these amino-1,3,5-triazine-based cross-linking agents, alkoxymethyl derivatives of melamine or guanamines are most notable.

Other well-known 1,3,5-triazine-derived cross-linking agents are carbamate-substituted 1,3,5-triazine cross-linking agents. For example, EP 0 814 104, US 4,708,984, and US 4,710,542 all pertain to 1,3,5-triazine compounds with the general structure C₃N₆(CH₂OR)₆₋ₓ(CH₂NHCOOR')ₓ, wherein R is hydrogen or C₁-C₁₂ alkyl, and R' is C₂-C₁₈ β-hydroxyalkyl or C₁-C₂₀ alkyl. It was shown that these carbamate-functionalised melamines yield coatings with resistance to detergent and salt-spray exposure and improved abrasion resistance. Since especially for automotive coatings, acid resistance is becoming an increasingly desirable property due to the increasing acidity of rain, alternative melamine-based cross-linking agents which provide improved acid etch resistance to the cured coating have been developed for coating compositions comprising polyols. Such an improved melamine-based cross-linking agent is for example described in US 5,792,866 or US 6,063,922. The disclosed cross-linking agent is an at least bis-carbamate-functionalised 1,3,5-triazine compound of the general formula 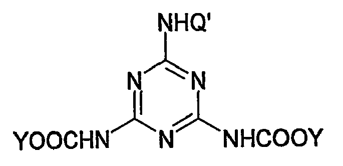 wherein Q' is a COOY moiety, a hydrocarbyl group or a hydrocarbyloxy hydrocarbyl ether group, and each Y is independently selected from the group consisting of a hydrocarbyl group and a hydrocarbyloxy hydrocarbyl ether group.

Curable compositions containing such 1,3,5-triazine carbamates are for example disclosed in US 5,852,133, US 5,593,735, or WO 98/18856. For instance, coating compositions containing a hydroxy-functional acrylic or polyester resin and a 1,3,5-triazine-2,4,6-tris-carbamate cross-linking agent are described. In practice, however, usually a mixture of cross-linking agents comprising a bis- or tris-carbamate-functionalised melamine-based cross-linking agent and a conventional melamine-formaldehyde (MF) cross-linking agent is used. Such coating compositions are known to exhibit excellent acid etch resistance.

However, a disadvantage of these bis- and tris-substituted melamine cross-linkers is the high degree of substitution with carbamate groups, resulting in complex and inefficient cross-linking processes, or at least in a lower cross-link density as compared with conventional MF cross-linkers. Furthermore, the cost price of these compounds is high compared with that of conventional melamine-formaldehyde (MF) resins. Another disadvantage of said bis- and tris-substituted melamine cross-linkers is the increased film hardness, often accompanied by brittleness, reducing resistance to impact, e.g. stone chip resistance. Furthermore, the bis- and especially the tris-carbamate melamine. cross-linkers show a tendency to crystallise or they exhibit poor compatibility in many coating formulations, thus increasing the haze of cured clear coat films.

Accordingly, there is a need for cross-linking agents for polyols which can be prepared in a relatively easy and efficient way at a low cost price, while yielding coatings with high film hardness, good polishability, good resistance to water and organic solvents, excellent durability, good miscibility with conventional coating compositions, and above all, good acid etch resistance.
We have surprisingly found that specific carbamate-melamine formaldehyde agents exhibit excellent cross-linking properties and, in addition, are readily accessible and commercially attractive because of their relatively low cost price.
More particularly, the invention pertains to carbamate-melamine formaldehyde cross-linking agents which are a mixture of 1,3,5-triazine compounds of the general formula: 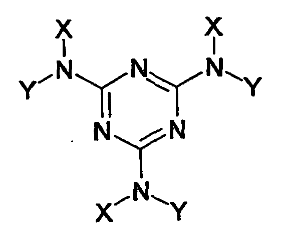 wherein
- X is selected from the group consisting of -COOR, -(CH₂-O)ₗ-H,
   -(CH₂-O)ₗ-R, -(CH₂-O)ₖ-CH₂-N(X)-Q, and -(CH₂-O)ₖ-CH₂-N(Y)-Q;
- Y is independently selected from the group consisting of -H, -(CH₂-O)ₗ-H,-(CH₂-O)ₗ-R, -(CH₂-O)ₖ-CH₂-N(X)-Q, and -(CH₂-O)ₖ-CH₂-N(Y)-Q;
- with each k independently being, on average, 0-10, preferably 1-5, more preferably 1 or 2, and most preferably about 1, and each I independently being, on average, 1-10, preferably 1-5, more preferably 1 or 2, and most preferably about 1;
- each R being independently selected from the group consisting of alkyl, cycloalkyl, and alkylaryl groups. Preferably, these R-groups contain fewer than 13 carbon atoms. More preferably, R is an alkyl group, even more preferably a linear alkyl group, preferably a C₁-C₈ alkyl group, and most preferably each R is a methyl group, a butyl group, or a mixture thereof,
- Q being 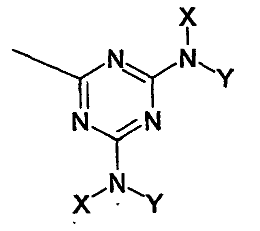
- and wherein said mixture of compounds comprises on average at least 0.05, but fewer than 2 carbamate groups per triazine moiety.

Preferably, the cross-linking agents according to the present invention comprise on average at least 0.1 carbamate group per triazine moiety, more preferably on average at least 0.25 carbamate groups, and most preferably on average at least 0.5 carbamate groups per triazine moiety of the melamine-derived cross-linking agent. Preferably, they comprise on average at most 1.9 carbamate groups per triazine moiety, more preferably on average at most 1.5 carbamate groups, even more preferably on average at most 1.2 carbamate groups, and most preferably on average at most 1.0 carbamate group per triazine moiety of the melamine-derived cross-linking agent.

If present, the total number of -(CH₂O)ₗ-H groups per triazine moiety on average preferably is at most 1.5, more preferably at most 0.8, and most preferably at most 0.5. The fact that methylol groups are present may mean that formaldehyde can be split off during curing of a final resin comprising the carbamate-melamine formaldehyde agents according to the invention. This can have advantages if a sag control agent is used which reacts with formaldehyde. Typically, such a sag control agent is a urea-based compound.

The preferred total number of -(CH₂O)ₗ-R groups per triazine moiety on average is at least 1.0, more preferably at least 1.5, and most preferably at least 2.0. The total number of -(CH₂O)ₗ-R groups present per triazine moiety on average preferably is at most 3.5, more preferably at most 3.0, and most preferably at most 2.5.

The degree of self-cross-linking of the carbamate-melamine formaldehyde cross-linking agents according to the present invention, resulting in a weight average number of triazine moieties per molecule, on average is more than 1. Preferably, the degree of self-cross-linking on average is between 1 and 200, more preferably between 1 and 50, even more preferably between 1 and 25, and most preferably between 1 and 15 per molecule. The weight average molecular weight (M_{w}) of said cross-linking agents is between 700 and 10,000. Preferably, the M_{w} is in the range of 850 and 7,500, more preferably in the range of 1,000 and 5,000, and most preferably in the range of 1,200 and 2,500. It is noted that in the carbamate-melamine formaldehyde cross-linking agents according to the present invention products comprising two carbamate groups per 1,3,5-triazine moiety may be present. These so-called bis-carbamates per each 1,3,5-triazine moiety preferably contain at least one tertiary amine group directly attached to said 1,3,5-triazine moiety.

In order to guarantee a storage stable product, suitable stabilisers can be added. Usually a pH controlled between 6.5 and 8.7 provides good stability. Preferably, the pH is controlled between 7 and 8.
The carbamate-melamine formaldehyde cross-linking agents according to the present invention can be prepared by any method known in the art. Preferably, however, an amine moiety of a 1,3,5-triazine compound is reacted with an organic carbonate, forming a carbamate group, after which the remaining primary amine moieties of the 1,3,5-triazine compound(s) are condensed with formaldehyde in the presence of an alcohol. Accordingly, the invention further pertains to the preparation of the carbamate-melamine formaldehyde cross-linking agents according to the present invention. Said process comprises the steps of
- mixing a 1,3,5-triazine compound and at least one base;
- adding at least one organic carbonate;
- neutralising the base by addition of an acid;
- removing the resulting salt by filtering and washing with water; and
- adding formaldehyde and at least one alcohol to form the corresponding methylol ethers.
Optionally, the thus obtained product may be distilled and/or filtrated before final use.

The 1,3,5-triazine compound preferably is melamine or a derivative thereof. The base used in the above-mentioned process can be any relatively strong base conventionally used in these kinds of processes. It is preferred that the base be of sufficient strength to deprotonate the amino groups of the chosen 1,3,5-triazine on reaction with the organic carbonate. Based upon the choice of starting 1,3,5-triazine, one of ordinary skill in the art will readily be able to determine which bases are capable of so deprotonating the amino groups of the 1,3,5-triazine compounds. As preferred examples of suitable bases may be mentioned alkali metal hydrides, alkali metal alkoxides, alkali metal hydroxides, alkali metal oxides, alkali metal carbonates, quaternary ammonium alkoxides, quaternary ammonium hydroxides, quaternary phosphonium alkoxides, quaternary phosphonium hydroxides, tertiary amines, and mixtures thereof. Sodium and potassium alkoxides are most preferred, and include linear, branched, and cyclic alkyl group-containing alkoxides and mixtures thereof. Particularly preferred bases include sodium hydride, potassium hydride, sodium butoxide, potassium butoxide, sodium methoxide, potassium methoxide, sodium ethoxide, potassium ethoxide, sodium propoxide, potassium propoxide, and mixtures thereof. It is also preferred that the base does not destroy the organic carbonate at a rate sufficiently high to compete with the reaction of the carbonate with the 1,3,5-triazine compound. In cases where the organic carbonate may be partially destroyed by the base, the use of excess amounts of the organic carbonate ensures a high conversion to the carbamate.

A wide variety of organic carbonates are suitable for use in the process of the present invention. Preferred organic carbonates are diaryl carbonates, dialkyl carbonates, aryl alkyl carbonates, cyclic carbonates, and dialkenyl carbonates. In preferred embodiments the organic carbonates are represented by the formula R(CO₃)R, wherein each R is independently selected from the group consisting of alkyl, cycloalkyl, and alkyl aryl groups. Preferably, the R-group contains fewer than 13 carbon atoms. More preferably, R is an alkyl group, even more preferably a linear alkyl group, preferably a C₁-C₈ alkyl group, and most preferably a methyl group, a butyl group, or a mixture thereof.
Examples of preferred dialkyl carbonates are dimethyl carbonate, diethyl carbonate, dipropyl carbonate, dibutyl carbonate, methyl butyl carbonate, and the like. Preferred aryl alkyl carbonates include methyl phenyl carbonate, butyl phenyl carbonate, and the like. Preferred diaryl carbonates include diphenyl carbonate, di(para-tolyl) carbonate, di(alpha-naphthyl) carbonate, di-(beta-naphthyl) carbonate, and the like. A preferred dialkenyl carbonate is diallyl carbonate. Examples of preferred cyclic carbonates are 1,2-ethanediol carbonate, 1,2-propanediol carbonate, and the like. Most preferably, the organic carbonate used in the process according to the present invention is selected from the group consisting of dimethyl carbonate, diethyl carbonate, dibutyl carbonate, methyl butyl carbonate, diphenyl carbonate, propylene carbonate, and mixtures thereof. A large number of these organic carbonates are generally commercially available and usable as such. They may also be prepared by methods well known in the art.

Suitable acids for neutralising the base include but are not limited to acids with a pKa value of less than 6, preferably with a pKa value of less than 4.5, and most preferably with a pKa value between 1 and 3.5. Preferred acids include sulphuric acid, formic acid, oxalic acid, phosphoric acid, hydrochloric acid, or mixtures thereof. Organic acids and ion exchange resins may optionally be utilised for the neutralisation.

Typically, the formaldehyde is introduced by supplying H₂CO or a source of H₂CO, such as paraformaldehyde, or solutions of formaldehyde in alcohol or water.

Suitable alcohols are compounds of the general formula ROH, wherein R is selected from the group consisting of alkyl, cycloalkyl, and alkyl aryl groups. Preferably, the R-group contains fewer than 13 carbon atoms. More preferably, R is an alkyl group, even more preferably a linear alkyl group, preferably a C₁₋C₈ alkyl group, and most preferably a methyl group, a butyl group, or a mixture thereof.

Preferably, the triazine compound and the base are mixed in a conventional kneader or other conventional mixing device. In the process of the present invention, the 1,3,5-triazine, the organic carbonate, and the base are contacted at a temperature and a length of time sufficient to produce the carbamate-melamine formaldehyde cross-linking agents according to the present invention. The components are preferably contacted at temperatures in the range of from about 0°C to about 150°C, and more preferably in the range of from about 25°C to about 100°C. At these temperatures and depending upon the starting components, reaction times ranging from about ten minutes to about ten hours are achieved.

An advantage of the process according to the present invention is that any remaining insoluble triazines or triazine (mono, di-)carbamates are converted into reactive, soluble products that are compatible with many existing organic resins used in coating compositions. Accordingly, said process results in fast reaction times, which make it possible to perform the preparation of these cross-linking agents at relatively low costs. In order to decrease the process costs even further, we have surprisingly found that the first process step, *i.e.* converting a 1,3,5-triazine compound into a carbamate triazine compound by reaction with an organic carbonate in the presence of a base, can be very efficiently carried out using a conventional extruder, yielding the intermediate product in very short process times and in a continuous manner.

Furthermore, the invention relates to a curable coating composition comprising at least one polyhydroxy group-containing polymer and the carbamate-melamine formaldehyde cross-linking agent. The invention also relates to the use of these curable compositions in coatings, such as general industrial coatings, automotive coatings, coil coatings, powder coatings, baked enamels, and wood finishes. The coatings, both solvent borne and water borne, are used in primer applications and top coat applications, including separate base and clear coat applications.
Besides a carbamate-melamine formaldehyde cross-linking agent according to the invention, curable coating compositions according to the present invention comprise at least one polyhydroxy group-containing polymer.

Said polyhydroxy group-containing polymer can be any polyhydroxy group-containing polymer conventionally used in amino resin coating compositions. Particularly suitable polymers include polyols, hydroxy-functional acrylic resins containing pendant or terminal hydroxy functionalities, hydroxy-functional polyester resins containing pendant or terminal hydroxy functionalities, hydroxy-functional polyurethanes, polyurethane-modified polyester polyols, polyurethane-modified polyacrylate polyols, and mixtures thereof. The polyhydroxy group-containing polymer contains on average at least two, and preferably more than two, hydroxy groups per molecule. Preferred polyhydroxy group-containing polymers are those with an acrylic or polyester backbone.

The polyhydroxy group-containing polymers preferably have weight average molecular weights of at least 1,000 to about 2,000,000. Usually, the weight average molecular weight is between 2,000 and 1,000,000, more preferably between 2,000 and 800,000, and most preferably between 2,500 and 100,000. The weight average molecular weight depends on the application requirements, and methods to modify the molecular weight accordingly are known to the skilled man.

Preferably, the coating composition comprises a polymer having a hydroxy number of 20 to 300 mg KOH/g polymer and more preferably of 60 to 200 mg KOH/g polymer. Good curing results are obtained with the hydroxy-containing polymers when an amount of cross-linker is used which corresponds to 0.5 to 2 moles of carbamate plus alkyl ether groups per mole of hydroxy groups, and preferably 0.5 to 1.2 moles of carbamate plus alkyl ether groups per mole of hydroxy group of the polymer.
Examples of commercially available and suitable polyhydroxy-containing polymers include, but are not limited to DORESCO® TA 39-21 acrylic resin (Dock Resins, Linden, N.J.), JONCRYL® 500 acrylic resin (S.C. Johnson & Sons, Racine, WI), ACRYLOID® AT-400 acrylic resin (Rohm & Haas, Philadelphia, PA), CYPLEX® 1531 polyester resin (Cytec Industries, West Paterson, NJ), CARGILL 3000 and 5776 polyester resins (Cargill, Minneapolis, MN), TONE® polyester resin (Union Carbide, Danbury, CT), K-FLEX® XM-2302 and XM-2306 resins (King Industries, Norwalk, CT), CHEMPOL® 11-1369 resin (Cook Composites and Polymers (Port Washington, WI), JONCRYL® 540 acrylic emulsion polymer (S.C. Johnson & Sons, Racine, WI), RHOPLEX® AC-1024 acrylic emulsion resin (Rohm & Haas, Philadelphia, PA), XC® 4005 water-reducible acrylic resin (Cytec Industries, West Paterson, NJ), CRYLCOAT® 3494 solid hydroxy-terminated polyester resin (UCB CHEMICALS USA, Smyrna, GA), RUCOTE® 101 polyester resin (Ruco Polymer, Hicksville, NY), JONCRYL® SCX- 800-A and SCX-800-B hydroxy functional solid acrylic resins (S.C. Johnson & Sons, Racine, WI), ALFTALAT® AN 745 hydroxy-functional polyester resin, a product of Hoechst Corporation, and the like. Furthermore, short oil alkyds such as Setal® 84 - XX- 70, saturated polyesters such as Setal® 1703 - XX - 75 and Setal® 1715 - VX - 74, water borne urethane modified polyesters such as Setal® 6071 - AQ - 45, thermosetting acrylic polymers such as Setalux® 1389 - VX - 60, Setalux® 1756 - W - 65, Setalux® 1757 - W - 70, Setalux® 1760 - VB - 64, Setalux® 1770 VS-70, and Setalux® 1795 - VX - 74, acrylic polyols such as Setalux® 1753 - XS - 65, non-aqueous dispersions such as Setalux® 1801 - SS - 53, acrylic microgels such as Setalux® 1850 - SS - 50, water borne thermosetting acrylic polymers such as Setalux® 6100 - GR - 74, as well as the corresponding polymers which have been modified with a sag control agent such as Setal® 91703 - SS - 53, Setal® 91715 - SS - 55, Setalux® 91389 - VX - 45, Setalux® 91756 - VS - 60, Setalux® 91757 - VX - 60, Setalux® 91760 - SS - 53, Setalux® 91795 - VX - 60 can be used.

Said coating compositions according to the invention after curing yield films with high film hardness, good polishability, good resistance to water and organic solvents, good durability, and good acid etch resistance. A major advantage of the cross-linking agents according to the present invention is that they provide films with homogeneously distributed cross-linking units, whereas the above-discussed bis- or tris-carbamate cross-linking agents when used in combination with conventional MF-cross-linking agents provide films with locally high or low concentrations of cross-linking units. A homogeneous distribution of the cross-linking units results in a film which comprises fewer defects after curing, has better solvent resistance, better chemical resistance such as acid resistence, homogeneous hardness, and better mechanical properties. When bis- or tris-carbamate cross-linking agents are used as the sole cross-linking agent, a lower cross-link density is obtained than when the cross-linking agents of the present invention are applied. A different cross-linking density gives rise to different mechanical and chemical properties.

The coating compositions may include a catalyst or a blocked catalyst to enhance the cure reaction. Suitable catalysts are well-known in the art and include, for example, *p*-toluene sulfonic acid, dinonyl naphthalene disulfonic acid, dodecyl-benzene sulfonic acid, phenyl acid phosphate, monobutyl maleate, butyl phosphate, blocked dodecyl-benzene sulfonic acid, and hydroxy phosphate ester.

The coating composition of the invention may optionally comprise a solvent, for while it can be utilised, for example, in the form of substantially solid powder or dispersion, it is often desirable that the composition is in a substantially liquid state. The solvent can be an organic solvent and/or water, depending on the solubility characteristics of the components. Examples of useful organic solvents include methyl ethyl ketone, methyl isobutyl ketone, *n*-amyl acetate, ethylene glycol butyl ether acetate, propylene glycol monomethyl ether acetate, xylene, *N*-methyl pyrrolidone, n-butane, n-butyl acetate, isopropyl alcohol, or blends of aromatic hydrocarbons. The liquid coating compositions can readily be prepared via methods and in relative amounts which are recognisable to those of ordinary skill in the art in the relevant field, depending on the particular end use chosen.

The coating compositions of the present invention may further optionally comprise a variety of additional ingredients. Depending on their end use, they may comprise well-known auxiliaries and additives typically used in the coatings industry including, for example, foam inhibitors, levelling aids, pigments, pigment dispersing aids, dyes, UV absorbers and other stabilising additives, and the like. These additives are well-known to those skilled in the relevant art and need not be discussed further.

The curable coating compositions according to the present invention have utility in coatings, such as general industrial coatings, automotive coatings, coil coatings, powder coatings, baked enamels, and wood finishes. They are also usable as moulding and adhesive compositions.

In a preferred embodiment of the invention, the coating composition of the invention is used as a pigmented coating composition or clear coat coating composition. In such a composition, solvent can be present in an amount of at least about 0.01 weight percent, based on the total weight of the composition, preferably of at least about 10 weight percent, and more preferably of at least about 30 weight percent. At most, the solvent may be present in an amount of about 99 weight percent based on the total weight of the composition, preferably of at most about 60 weight percent, and more preferably of at most about 50 weight percent.
In a particularly preferred embodiment, the composition of the invention is used as a clear and/or colourless coating composition over a pigmented base coat as part of a composite colour-plus-clear coating. Such composite coatings are popular for their depth of colour and liquid glossy surface appearance. They have found particularly wide acceptance in the field of automotive coatings. The composition of the invention can also be used as the base coat of a composite colour-plus-clear coating.

The coating compositions of the present invention can be coated on the article by any of a number of techniques well-known in the art. These include, for example, spray coating, dip coating, roll coating, curtain coating, and the like.
For automotive body panels, spray coating is preferred. After contacting the substrate and the coating composition, the solvent is allowed to partially evaporate to produce a uniform coating on the substrate. Subsequently, the compositions can be subjected to conditions to cure the coating layers.

Although various methods of curing may be used, heat curing is preferred. Generally, heat curing is effected by exposing the coated article to elevated temperatures provided primarily by radiative heat sources. Curing temperatures will vary depending on the aminoplast and functional polymer used; however, they generally range between 70°C and 190°C and are preferably between 110°C and 160°C, most preferably between 125°C and 150°C. The curing time will vary depending on the particular components used and on physical parameters such as the thickness of the layers; however, typical curing times range from 15 to 60 minutes.

The present invention is elucidated by means of the following Examples.

Gloss was measured in a conventional way using a Dr. Lange gloss meter LMG 070 or a Byk Haze-Gloss 4601 gloss meter (reference is made to ISO 2813) and is expressed in Gloss units (GU).

### Example 1

1 mole of melamine was added to 15 moles of dimethyl carbonate. A strong base (NaOMe) was used to activate the triazines. The amount of carbonate groups formed on the melamine moiety is proportional to the concentration of the base. The reaction was carried out under stirring at 70°C. The reaction product was isolated and dried. Unreacted dimethyl carbonate was recycled. The thus obtained intermediate product was added to 10 moles of butanol containing sufficient sulfuric acid to neutralise the strong base. Formic acid or sodium hydroxide was used to adjust the pH value of the slurry until a value between 6 and 6.5 was obtained. Subsequently, 5 moles of formaldehyde were added and the reaction mixture was heated until reflux conditions were obtained. After 4 hours, the pH value was raised by adding enough sodium hydroxide to obtain a pH value of 7 - 7.5. Excess butanol was distilled off. The carbamate content was checked using IR spectroscopy and from this a carbamate group content of 0.65 ± 0.1 per triazine moiety was found. NMR results yielded a carbamate content of more than 0.4 per triazine moiety. The weight average molecular weight was 844 D as determined by GPC (Gel Permeation Chromatography).
The obtained product was used to produce a clear coat paint formulation. This was made using 53.3% of Setalux® 1760 VB-64 (ex Akzo Nobel Resins), 12.4 wt% of the just mentioned carbamate-melamine formaldehyde cross-linking agent (based on 100% solids), 0.2 wt% Baysilon® 01-17 (ex Bayer), and 1.3% Nacure® 5225 (ex King Industries), in butanol/butyl glycol acetate. The final solids content of the paint was 48%. A layer was coated and cured at 140°C for 24 minutes. The appearance and the colour were good. The panels were evaluated for xylene resistance (*i.e.* a cross-link functionality check). After 5 minutes exposure time neither softening nor lifting was observed. The solvent resistance was tested by putting a droplet of MEK (methyl ethyl ketone) onto the surface of the coating and inspecting the panel after 5 minutes (20°C): there was some softening, but no lifting of the coating was observed.
The acid etch resistance was tested by adding an aliquot of sulfuric acid (10%) to the surface of the coating each minute for 20 minutes at 65°C. The panel was visually inspected after the last droplet was put onto the coating. Even after an exposure of 20 minutes after the first droplet was put onto the coating, no damage was observed in the clear coat and the gloss remained unaffected. The underlying base coat was also unaffected after 20 minutes exposure.

### Comparative Example 2

A layer was coated in a manner similar to that described in Example 1. However, now a paint formulation was used in which the carbamate-melamine formaldehyde cross-linking agent according to the present invention was replaced by an equal amount of conventional MF resin: viz. Setamine® US-138 BB-70 ex Akzo Nobel. In this case, the curing catalyst Nacure® 5225 was not employed.
The acid etch resistance was tested as described in Example 1. In the acid etch test, a stain of the acid etch was observed after 11 minutes, resulting in significant loss of gloss. The underlying base coat was visually affected after 14 minutes of acid etch.
The solvent resistance was again tested by putting a droplet of MEK (methyl ethyl ketone) onto the surface of the coating and inspecting the panel after 5 minutes (20°C): there was no softening, but moderate lifting of the coating was observed.

### Example 3

A layer was coated in a manner similar to that described in Example 1. However, instead of 10 moles of butanol 8 moles of butanol were used. The weight average molecular weight (M_{w}) of the resulting product was 2,190 D as determined by GPC. The carbamate content was, on average, 0.65 as determined by IR. A clear paint formulation comprising said product was prepared according to the procedure described in Example 1.
The MEK resistance test was carried out as described in Examples 1 and 2 and resulted in no softening and hardly any lifting. The acid etch resistance was tested as described in Example 1. The staining of the underlying base coat was inspected: only after 20 minutes did a slight acid stain become visible.

### Comparative example 4

A similar paint formulation to that described in Example 1 was prepared, wherein Cylink 2000 (ex Cytec) was used as the cross-linking agent (*i.e.* 100% of the cross-linker used consisted of Cylink 2000), instead of the carbamate-melamine formaldehyde cross-linking agent according to the invention.

The solvent resistance was tested by putting a droplet of MEK (methyl ethyl ketone) onto the surface of the coating and inspecting the panel after 5 minutes (20°C): some softening and a moderate lifting of the coating were observed.

### Example 5

To 1 mole of melamine was added a solution of 1.5 moles of NaOMe in methanol. The resulting mixture was heated to 90°C and stirred vigorously. Subsequently, 1.5 moles of dimethylcarbonate were added to the reactor. After a reaction time of 4 hours, the reaction mixture was cooled to 40°C, the product was neutralised with formic acid, and the resulting salt removed by filtration and washing with water. The isolated product was dried and subsequently 10 moles of butanol and 5.7 moles of formaldehyde were added. The reaction mixture was subsequently heated to reflux temperature. The pH was adjusted during the process and maintained between 4 and 5. A mixture of butanol, water, and methanol was distilled off and once the reaction mixture was clear, the pH was adjusted to 7 ± 0.5 and the product filtered. The final product was clear and colourless and had a solids content of 50.2%.
The carbamate content was checked using IR spectroscopy and from this a carbamate group content of, on average, 1.3 ± 0.2 per triazine moiety was found. The weight average molecular weight (M_{w}) was 1,770 D as determined by GPC.
The obtained product was used for the preparation of a clear coat paint formulation. Said formulation was made using 98.8 pbw (parts by weight) of Setalux® 1795-VX-74 (ex Akzo Nobel Resins), 53.6 pbw of the just mentioned carbamate-melamine formaldehyde cross-linking agent, 5.0 pbw of Baysilon® OI-17 (ex Bayer), and 2.8 pbw of Nacure® 5225, in butanol/butyl glycol acetate. The final solids content of the paint was 50.9% at a viscosity of 24 sec DIN 4. A layer was coated and cured at 140°C for 24 minutes.
The appearance and colour were good. A gloss of 90 GU (20°) was measured. An acid etch test was performed as described in Example 1. The staining of the underlying base coat was inspected but even after 30 minutes no stain was visible.

### Comparative example 6

A layer was coated in a manner similar to that described in Example 5. However, now a paint formulation was used wherein the carbamate-melamine formaldehyde cross-linking agent according to the present invention was replaced by an equal amount (calculated on solids) of a conventional MF resin, *viz.* Setamine® US-138 BB-70 ex Akzo Nobel. In this case, the curing catalyst Nacure® 5225 was not employed.
A gloss of 92 GU (20°) was measured. In the acid etch test (see Example 1), the underlying base coat was visually inspected and appeared to be affected after 16 minutes, resulting in a significant loss of gloss. Total loss of gloss was observed after 17 minutes exposure time.

### Comparative example 7

A layer was coated in a manner similar to that described in Example 6. However, the paint formulation used was the one described in Example 6 to which an amount of Nacure® 5225 was added equal to the amount used in Example 5. The appearance and colour of the cured coating were good. In the acid etch test (see Example 1) the underlying base coat was visually inspected and appeared to be affected after 16 minutes, resulting in a significant loss of gloss. Total loss of gloss was observed after 20 minutes exposure time.

### Example 8

A cross-linking agent was prepared following the procedure according to Example 5 but now using a melamine:dimethylcarbonate:sodium methoxide ratio of 1:1:1, and using oxalic acid to neutralise the intermediate product. The final carbamate-melamine formaldehyde cross-linking agent was prepared starting from said intermediate product by bringing it into contact with formaldehyde and butanol with the butanol:melamine ratio being 20:1 and the formaldehyde:melamine ratio being 4.6:1. The end product was distilled to a solids content of 57.8%. A weight average molecular weight (M_{w}) of 1,241 D was obtained as determined by GPC. In this case, a carbamate content of, on average, 0.45 was obtained as determined by IR.
A layer was coated with a paint formulation similar to the one described in Example 5, but wherein the acrylic polyalcohol Setalux® 1795-VX-74 (ex Akzo Nobel Resins) was replaced by Setalux® 1770-VS-70 (ex Akzo Nobel Resins) and wherein the carbamate-melamine formaldehyde cross-linking agent of Example 5 was replaced by the just described carbamate-melamine formaldehyde cross-linking agent.

In the same evaluation experiment a reference coating was included with a similar composition to the one just described, but wherein the carbamate-melamine formaldehyde cross-linking agent was replaced (1 on 1, calculated on solids) by a conventional MF resin, viz. Setamine® US-138 ex Akzo Nobel and wherein the addition of 2.8% pbw Nacure® 5225 was omitted.

In an acid etch test performed as mentioned in Example 1, the clear coat and the underlying base coat of both panels were visually inspected.
After 11 minutes, the first damage to the clear coat comprising the carbamate-melamine formaldehyde cross-linking agent was observed, whereas for the reference sample the first damage was observed after 7 minutes. First damage to the underlying base coat was observed after 18 minutes, whereas for the reference sample first damage already became visible after 13 minutes. After 20 minutes total loss of gloss was observed, compared to 18 minutes for the reference sample.

### Example 9

A carbamate triazine compound was prepared according to the following procedure in an extruder, viz. a Leistritz Micro18 co-rotating twin-screw extruder. The extruder consisted of 8 heated zones (7 houses and the die head) and a water-cooled feeding barrel. The extruder was equipped with a set of modular screws wherein the closely intermeshing screws had a diameter of 18 mm and L/D=40. The design of the screws allowed solid feed in house 1 and dosing of liquids in houses 2, 4, and 7. The die of the extruder was a bent tube with a length of 35 mm and an inner diameter of 4 mm.
Solid melamine was fed in the water-cooled feeding zone. NaOMe in a methanol solution and DMC were dosed in the first heated house (*i.e.* house 2) by means of a peristaltic pump and a membrane pump, respectively. The reaction temperature was kept at 90 °C. The die head was kept at 60°C to prevent boiling of the methanol when the extrudate was discharged from the extruder. The process time for this step was about 5 minutes.
The composition of the resulting product was analysed by means of FTIR and HPLC (UV-detection) to prove the formation of carbamate-functional melamines.

### Temperature settings during reactive extrusion

| measured zone temperatures | | | | Melt temperature (°C) | die pressure (bar) |
|---|---|---|---|---|---|
| 5 6 (°C) | (°C) | h7 (°C) | 8 (die) (°C) | | |
| 75 | 90 | 78 | 61 | 74 | 2.5-2.8 |

### Composition of the sample (feedstock)

| Experiment | Throughputs | | | | | |
|---|---|---|---|---|---|---|
| | melamine (g/h) | NaOMe (g/h) | DMC (g/h) | melamine (mol/h) | NaOMe (mol/h) | DMC (mol/h) |
| Product | 250 | 712 | 349 | 1.98 | 3.96 | 3.87 |

The amount of carbamate groups per triazine moiety was found to be, on average, 0.6.
The resulting reaction mixture was used to make a carbamate MF resin according to the procedure shown in Example 1, which gave good acid etch performance in cured coating compositions.

## Claims

1. A mixture of 1,3,5-triazine compounds of the general formula: 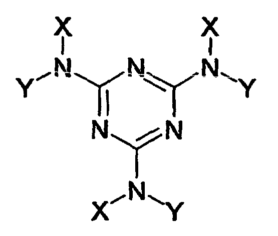 wherein
- X is selected from the group consisting of -COOR, -(CH₂-O)ₗ-H,
-(CH₂-O)ₗ-R, -(CH₂-O)ₖ-CH₂-N(X)-Q, and -(CH₂-O)ₖ-CH₂-N(Y)-Q;
- Y is independently selected from the group consisting of -H,
-(CH₂-O)ₗ-H, -(CH₂-O)ₗ-R, -(CH2-O)ₖ-CH₂-N(X)-Q, and
-(CH₂-O)ₖ-CH₂-N(Y)-Q;
- with each k independently being, on average, 0-10, and each I independently being, on average, 1-10;
- each R being independently selected from the group consisting of alkyl, cycloalkyl, and alkyl aryl groups;
- Q being 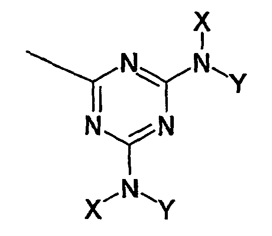
and
wherein said mixture of compounds comprises on average at least 0.05, but fewer than 2 carbamate groups per triazine moiety.

2. A mixture of 1,3,5-triazine compounds according to claim 1, **characterised in that** k and l are each independently, on average, 0.5-2.5, preferably about 1.

3. A mixture of 1,3,5-triazine compounds according to claim 1 or 2, **characterised in that** R is a methyl group or a butyl group or a mixture thereof.

4. A mixture of 1,3,5-triazine compounds according to any one of claims 1-3, **characterised in that** the total number of -(CH₂O)ₗ-H groups per triazine moiety on average is at most 1.5, more preferably at most 0.8, whereas the total number of -(CH₂O)ₗ-R groups per triazine moiety on average is at least 1.0 and at most 3.5.

5. A mixture of 1,3,5-triazine compounds according to any one of claims 1-4, **characterised in that** it comprises on average at least 0.1, preferably on average at least 0.25, and most preferably on average at least 0.5 carbamate groups per triazine moiety, and on average at most 1.9, preferably on average at most 1.5, even more preferably on average at most 1.2, and most preferably on average at most 1.0 carbamate groups per triazine moiety.

6. A coating composition comprising
a) at least one polyhydroxyl group-containing polymer,
and
b) the mixture of 1,3,5-triazine compounds according to any one of claims 1-5.

7. A coating composition according to claim 6, **characterised in that** the polyhydroxyl group-containing polymer is selected from the group of acrylic polyols, polyester polyols, and polyurethane polyols.

8. A coating composition according to claim 6 or 7, **characterised in that** the coating composition further comprises one or more additives selected from the group consisting of curing catalyst, solvent, foam inhibitor, levelling aid, pigment, pigment dispersing aid, dye, and UV absorber.

9. Use of the coating composition according to any one of claims 6-8 in general industrial coatings, automotive coatings, coil coatings, powder coatings, baked enamels, or wood finishes.

10. Use of the coating composition according to any one of claims 6-8 in the field of finishing and refinishing of automobiles and large transportation vehicles.

11. A process for the preparation of a mixture of 1,3,5-triazine compounds according to any one of claims 1-5, comprising the following steps:
- mixing a 1,3,5-triazine compound and at least one base;
- adding at least one organic carbonate;
- neutralising the base by addition of an acid;
- removing the resulting salt by filtering and washing with water; and
- adding formaldehyde and an alcohol to form the corresponding methylol ethers.

12. A process according to claim 11 wherein the mixing of the 1,3,5-triazine compound and the base is carried out using an extruder.

## Patentansprüche

1. Mischung von 1,3,5-Triazinverbindungen der allgemeinen Formel: 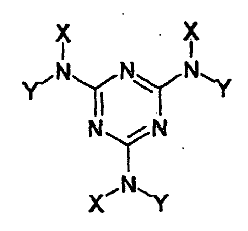 wobei
- X aus der aus -COOR, -(CH₂-O)ₗ-H, -(CH₂-O)ₗ-R, -(CH₂-O)ₖ-CH₂-N(X)-Q und -(CH₂-O)ₖ-CH₂-N(Y)-Q bestehenden Gruppe ausgewählt ist;
- Y unabhängig aus der aus -H, -(CH₂-O)ₗ-H, -(CH₂-O)ₗ-R, -(CH₂-O)ₖ-CH₂-N(X)-Q und -(CH₂-O)ₖ-CH₂-N(Y)-Q bestehenden Gruppe ausgewählt ist;
- wobei jedes k unabhängig durchschnittlich 0 - 10 ist und jedes I unabhängig durchschnittlich 1 - 10 ist;
- wobei jedes R unabhängig aus der aus Alkyl-, Cycloalkyl- und Alkylarylgruppen bestehenden Gruppe ausgewählt ist;
- Q 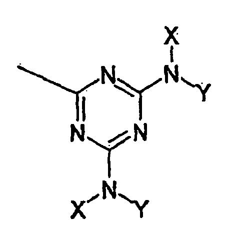
ist und
wobei die Mischung von Verbindungen durchschnittlich wenigstens 0,05, aber weniger als 2 Carbamatgruppen pro Triazinrest umfasst.

2. Mischung von 1,3,5-Triazinverbindungen nach Anspruch 1, **dadurch gekennzeichnet, dass** k und l jeweils unabhängig durchschnittlich 0,5 - 2,5, vorzugsweise etwa 1 sind.

3. Mischung von 1,3,5-Triazinverbindungen nach Anspruch 1 oder 2, **dadurch gekennzeichnet, dass** R eine Methylgruppe oder eine Butylgruppe oder eine Mischung davon ist.

4. Mischung von 1,3,5-Triazinverbindungen nach einem der Ansprüche 1 - 3, **dadurch gekennzeichnet, dass** die Gesamtzahl der -(CH₂O)ₗ-H-Gruppen pro Triazinrest durchschnittlich höchstens 1,5, noch mehr bevorzugt höchstens 0,8 beträgt, während die Gesamtzahl der -(CH₂O)ₗ-R-Gruppen pro Triazinrest durchschnittlich wenigstens 1,0 und höchstens 3,5 beträgt.

5. Mischung von 1,3,5-Triazinverbindungen nach einem der Ansprüche 1 - 4, **dadurch gekennzeichnet, dass** sie durchschnittlich wenigstens 0,1, vorzugsweise durchschnittlich wenigstens 0,25 und am meisten bevorzugt durchschnittlich wenigstens 0,5 Carbamatgruppen pro Triazinrest und durchschnittlich höchstens 1,9, vorzugsweise durchschnittlich höchstens 1,5, sogar noch mehr bevorzugt durchschnittlich höchstens 1,2 und am meisten bevorzugt durchschnittlich höchstens 1,0 Carbamatgruppen pro Triazinrest umfasst.

6. Beschichtungszusammensetzung, umfassend
a) wenigstens ein Polyhydroxylgruppen enthaltendes Polymer und
b) die Mischung von 1,3,5-Triazinverbindungen nach einem der Ansprüche 1 - 5.

7. Beschichtungszusammensetzung nach Anspruch 6, **dadurch gekennzeichnet, dass** das Polyhydroxylgruppen enthaltende Polymer aus der aus Acrylpolyolen, Polyesterpolyolen und Polyurethanpolyolen bestehenden Gruppe ausgewählt ist.

8. Beschichtungszusammensetzung nach Anspruch 6 oder 7, **dadurch gekennzeichnet, dass** die Beschichtungszusammensetzung weiterhin ein oder mehrere Additive umfasst, die aus der aus einem Härtungskatalysator, Lösungsmittel, Schaumverhinderungsmittel, Verlaufmittel, Pigment, Pigment-Dispergierhilfsmittel, Farbstoff und einem UV-Absorptionsmittel bestehenden Gruppe ausgewählt sind.

9. Verwendung der Beschichtungszusammensetzung nach einem der Ansprüche 6 - 8 in allgemeinen Industriebeschichtungen, Kraftfahrzeugbeschichtungen, Wicklungsbeschichtungen, Pulverbeschichtungen, gebrannten Emaille oder Holz-Decklacken.

10. Verwendung der Beschichtungszusammensetzung nach einem der Ansprüche 6 - 8 auf dem Gebiet der Decklackierung und Reparaturlackierung von Kraftfahrzeugen und großen Transportfahrzeugen.

11. Verfahren zur Herstellung einer Mischung von 1,3,5-Triazinverbindungen nach einem der Ansprüche 1 - 5, umfassend die folgenden Schritte:
- Vermischen einer 1,3,5-Triazinverbindung mit wenigstens einer Base;
- Zugabe wenigstens eines organischen Carbonats;
- Neutralisieren der Base durch die Zugabe einer Säure;
- Entfernen des resultierenden Salzes durch Filtrieren und Waschen mit Wasser und
- Zugabe von Formaldehyd und einem Alkohol zur Bildung der entsprechenden Methylolether.

12. Verfahren nach Anspruch 11, wobei das Vermischen der 1,3,5-Triazinverbindung und der Base in einem Extruder erfolgt.

## Revendications

1. Mélange de composés 1,3,5-triazine de formule générale : 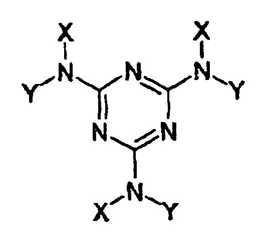 où
- X est choisi à partir du groupe comportant -COOR, -(CH₂-O)ₗ-H, -(CH₂-O)ₗ-R, - (CH₂-O)ₖ-CH₂-N (X) -Q, et -(CH₂-O)ₖ-CH₂-N(Y)-Q;
- Y est indépendamment choisi à partir du groupe comportant -H, -(CH₂-O)ₗ-H, -(CH₂-O)ₗ-R, -(CH₂-O)ₖ-CH₂₋N(X)-Q, et -(CH₂-O)ₖ-CH₂-N(Y)-Q ;
- chaque k valant indépendamment, en moyenne, de 0 à 10, et chaque 1 valant indépendamment, en moyenne, de 1 à 10 ;
- chaque R étant indépendamment choisi à partir du groupe comportant des groupes alkyle, cycloalkyle et alkyle aryle ;
- Q étant 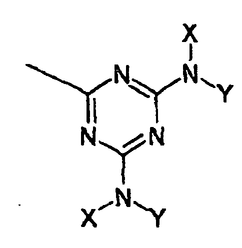
et
où ledit mélange de composés comprend en moyenne au moins 0,05, mais moins de 2 groupes carbamate par fraction de triazine.

2. Mélange de composés 1,3,5-triazine selon la revendication 1, **caractérisé en ce que** k et 1 valent chacun indépendamment, en moyenne, de 0,5 à 2,5, de préférence environ 1.

3. Mélange de composés 1,3,5-triazine selon la revendication 1 ou 2, **caractérisé en ce que** R est un groupe méthyle ou un groupe butyle ou un mélange de ceux-ci.

4. Mélange de composés 1,3,5-triazine selon l'une quelconque des revendications 1 à 3, **caractérisé en ce que** le nombre total de groupes -(CH₂O)ₗ-H par fraction de triazine en moyenne est 1,5 au plus, de préférence 0,8 au plus, alors que le nombre total de groupes -(CH₂O)ₗ-R par fraction de triazine en moyenne est au moins 1,0 et au plus 3, 5.

5. Mélange de composés 1,3,5-triazine selon l'une quelconque des revendications 1 à 4, **caractérisé en ce qu'**il comprend en moyenne au moins 0,1, de préférence en moyenne au moins 0,25, et de manière préférée entre toutes en moyenne au moins 0,5 groupe(s) carbamate par fraction de triazine, et en moyenne au plus 1,9, de préférence en moyenne au plus 1,5, de préférence en moyenne au plus 1,2, et de manière préférée entre toutes en moyenne au plus 1,0 groupe(s) carbamate par fraction de triazine.

6. Composition de revêtement comprenant
a) au moins un polymère contenant des groupes polyhydroxyle
et
b) le mélange de composés 1,3,5-triazine selon l'une quelconque des revendications 1 à 5.

7. Composition de revêtement selon la revendication 6, **caractérisée en ce que** le polymère contenant des groupes polyhydroxyle est choisi à partir du groupe de polyols acryliques, de polyols polyester et de polyols de polyuréthane.

8. Composition de revêtement selon la revendication 6 ou 7, **caractérisée en ce que** la composition de revêtement comprend en outre un ou plusieurs additifs choisi à partir du groupe comportant un catalyseur de vulcanisation, un solvant, un anti-mousse, un agent facilitant l'étalement, un pigment, un agent facilitant la dispersion du pigment, un colorant et un anti-UV.

9. Utilisation de la composition de revêtement selon l'une quelconque des revendications 6 à 8 dans les revêtements utilisés dans l'industrie générale, les revêtements utilisés dans l'industrie automobile, les prélaquages en continu, les revêtements en poudre, les émaux au four, ou les finitions du bois.

10. Utilisation de la composition de revêtement selon l'une quelconque des revendications 6 à 8 dans le domaine du finissage et du revernissage dans l'industrie de l'automobile et des gros véhicules de transport.

11. Processus de préparation d'un mélange de composés 1,3,5-triazine selon l'une quelconque des revendications 1 à 5, comprenant les étapes suivantes consistant à :
- mélanger un composé 1,3,5-triazine et au moins une base ;
- ajouter au moins un carbonate organique ;
- neutraliser la base par addition d'un acide ;
- éliminer le sel résultant en filtrant et en lavant avec de l'eau ;
et
- ajouter du formaldéhyde et un alcool pour former les éthers de méthylol correspondants.

12. Processus selon la revendication 11 dans lequel le mélange du composé 1,3,5-triazine et de la base est effectué à l'aide d'une extrudeuse.
